# EUROPÄISCHE PATENTSCHRIFT

(11) **EP 3 243 523 B1**
(45) Veröffentlichungstag und Bekanntmachung des Hinweises auf die Patenterteilung: **29.04.2020**
(21) Anmeldenummer: 17000802.3
(22) Anmeldetag: 10.05.2017
(51) Int. Cl.: A61K 38/16, A61P 35/04, A61P 35/00, A61K 9/107

(54) **LEKTINE UND KREBSPROPHYLAXE**
LECTINS AND CANCER PROPHYLAXIS
LECTINE ET PROPHYLAXIE DU CANCER

(30) Priorität: 11.05.2016 EP 16001060
(43) Veröffentlichungstag der Anmeldung: 15.11.2017
(73) Patentinhaber: YACARE GmbH, 79261 Gutach (DE)
(72) Erfinder: Müller, Georg, D-79263 Simonswald (DE)
(74) Vertreter: Helbig, Christian

(56) Entgegenhaltungen:
- EP-A2- 0 602 686
- EP-B1- 0 942 741
- WO-A1-99/27947
- DE-A1- 4 221 836
- US-A- 5 053 386
- US-A1- 2006 148 681

## Beschreibung

Die Erfindung betrifft eine pharmazeutische Formulierung sowie Ihre Verwendung zur Prophylaxe bzw. Verminderung oder Vermeidung von Metastasen bel Krebspatienten.

Krebs ist In den Industrialisierten Ländern eine der häufigsten Todesursachen. Hierbei Ist das Krankheitsbild nicht nur durch den unmittelbaren Befall von bestimmten Zellen oder Geweben gekennzeichnet, sondern die sekundäre Metastasenbildung bzw. die Auswanderung der primären Krebszellen In sekundäre Gewebe steht einer Hellung der Patienten entgegen.

Krebserkrankungen werden unterteilt in systemische Krebserkrankungen, z. B. Leukämien, Lymphome sowie In solide Krebserkrankungen, z. B. Karzinome, Sarkome, neuroendokrine Tumore. Dabei ist die Gruppe der Karzinome (z. B. Brust-, Darm-, Prostata-, Lungenkrebs) die mit Abstand häufigste Krebserkrankungsart und macht > 90% aller Krebserkrankungen aus. Laut Robert Koch Institut (Information vom 17. 12. 2015) werden für das Jahr 2016 500.000 neue Erkrankungen prognostiziert.

Lektine sind zuckerbindende Eiweiße mit hoher Spezifität für definierte Zucker und sie werden entsprechend, z. B, in der Analytik und Forschung, für die Bestimmung der Kohlenhydratantelle von Glykoproteinen eingesetzt.

EP 0 942 741 B1 beschreibt Lektine und Ihre Verwendung zur Kontrolle der Schleimhautzellprollferation. Die erfindungsgemäße Verwendung wird in diesem Dokument weder beschrieben noch nahegelegt.

Die Therapie von Krebserkrankungen erfolgt gemäß Internationalen Leitlinien. Der primäre Therapieerfolg wird eingeschränkt durch deren Rezldivlerung und Insbesondere durch deren Metastasierung. Karzinome metastasieren bevorzugt In Lunge und Leber, wodurch die Lebenserwartung der betroffenen Patienten/innen signifikant eingeschränkt wird.

Somit Ist ein bisher nicht hinreichend gelöstes Problem bei der Krebsbehandlung die Metastasenbildung und -ausbreitung zufriedenstellend zu behandeln bzw, zu vermelden oder zu vermindern und somit die Lebenserwartung der Patelnten/Innen zu erhöhen.

US2006/148681 erwähnt Zubereitungen enthaltend Concavalin A, *Pisum sativum* Agglutinin oder *Phaseolus vulgaris* Leucoagglutinin zur Behandlung von Tumoren sowie Verhinderung von Metastasen und Rückfällen.

Die der Erfindung zugrunde Ilegende Aufgabe bestand nun darin eine Behandlungsmethode zu finden, die nicht nur den Krebs In seiner Primärform positiv beeinflussen kann, sondern auch die Metastasenbildung vermindern hilft oder sie ganz verhindert. Eine weitere Aufgabe besteht darin die Metastasenbildung zu adressieren. Eine weitere Aufgabe bestand darin ein Mittel zu finden, das die Immunabwehr Induziert oder/und steigert und so den Organismus bel seinem Kampf gegen den Krebs endogen unterstützt. Im weiteren war es eine Aufgabe der Erfindung eine Behandlung von Krebs bereitzustellen, die sich durch geringere Nachtelle im Vergleich des Standes der Technik auszeichnet.

Diese Aufgabe wird gelöst durch eine pharmazeutische Formulierung umfassend ein oder mehrere Lektine zur Verwendung bel der Prophylaxe oder/und der Verhinderung oder Verminderung der Metastasenbildung bel Krebspatienten oder/und zur Steigerung oder Induzierung der Immunabwehr oder/und zur Immunogenislerung von Krebszellen, wobei der Krebs ein Karzinom ist, wobei das Lektin ein Erbsenlektin oder/und ein Linsenlektin ist, wobei das oder die Lektine in mizellislerter Form vorliegen.

Die erfindungsgemäße pharmazeutische Formulierung ist besonders vorteilhaft verwendbar bei Karzinomen, da hier eine starke Metastasierung auftritt. Karzinome sind z. B. Brust-, Darm-, Prostata- oder Lungenkarzinome.

Unter "pharmazeutische Formulierung" oder "Formulierung" Im Sinne der Erfindung Ist eine Zubereitung zu verstehen, die als Arzneimittel oder zur Anwendung als Nahrungsergänzungsmittel oder z.B. zur ergänzenden bilanzierten Dlät Anwendung finden kann. Hierbei kann die erfindungsgemäße Formulierung auch weitere und dem Fachmann bekannten Inhalts- oder/und Trägerstoffe beinhalten, die üblich und nützlich zur Zubereitung derartiger Formulierungen sind.

Dabei kann durch die Erfindung überraschender Weise erreicht werden, dass die Metastasenbildung vorteilhaft beeinflusst wird. Dadurch kann die primäre Krebstherapie weiter verbessert werden und die Lebensqualität sowie die Lebensdauer der Patienten erhöht werden. Eine effektive Metastasenprophylaxe ist weiterhin ein Aspekt der Erfindung und demnach eine Möglichkeit, eine bekannte Krebstherapie zu optimieren.

Hierbei Ist der Begriff "Krebsprophylaxe" wie folgt zu verstehen: 1. primäre Prophylaxe bedeutet Vorbeugemaßnahmen für gesunde Menschen, 2. sekundäre Prophylaxe bedeutet Früherkennungsmaßnahmen bzw. Vorsorge und 3, tertiäre Prophylaxe sind Vorbeugemaßnahmen nach erfolgter Krebstherapie. Die Erfindung Ist für alle drei Bereiche anwendbar, wobei die Erfindung Insbesondere für die tertiäre Prophylaxe wie oben definiert vorteilhaft ist.

Die Erfindung kann dabei wie folgt zur Anwendung kommen und macht sich die weiter unten beschriebenen wissenschaftlichen Grundlagen zu Nutze,

1. Primäre Prophylaxe, Grundlage Ist, dass In einem menschlichen Organismus regelmäßig Zellen entarten und unter gewissen Voraussetzungen (z. B. Abwehrschwäche In Stresssituationen oder als Alterserscheinung) zur Krebserkrankung werden.
Ein Problem von Krebserkrankungen Ist, dass Insbesondere Karzinomzellen oft kein erkennbares proteinhaltiges Antigen auf Ihrer Oberfläche präsentieren und demnach vom körpereigenen Abwehrsystem nicht erkannt werden können. Es sind aber sehr spezifische Kohlenhydratketten In den Karelnomzellmembranen präsentiert. Die Erfinder haben nun erkannt, dass eine probate Prophylaxe sein kann, an die Kohlenhydratantigene ein großmolekulares Agens anzudocken. Hierbei setzt die Erfindung Lektine In Ihrer Formulierung ein, vorzugsweise Linsenlektin.

In einem Aspekt, der die Aufnahme an sich oder eine verbesserte Aufnahme der Lektine betrifft wird das Lektin In einer optimierten mizellisierten Form verwendet. Dadurch kann eine verbesserte Resorption erreicht werden.

Das oder die Lektine liegen in der erfindungsgemäßen Formulierung in mizellislerter Form vor, vorzugsweise liegen sie als emulglerte Lektine vor, besonders bevorzugt als flüssige, wässrige, mizellare Emulsion oder als flüssige, wässrig-ötige, mizellare Emulsion. Vorzugsweise liegt das emulgierte Lektin in Vesikeln, Mischmizellen, als Mikroemulsion, als nanodisperses System oder Nanoemulsion vor, Besonders bevorzugt haben die Mizellen einen mittleren Durchmesser von 3 bis 300nm, vorzugsweise von 5 bis 250nm, besonders bevorzugt von 10 bis 120nm, mehr bevorzugt von 50 bis 80nm. Weiterhin Ist bevorzugt, dass die Emulsion der Lektinformullerung einen pH von 4 bis 9,5, vorzugsweise von 6 bis 8,5, vorzugsweise von 8,5 bis 9,5 aufweist.

Vorzugsweise sind weiteren Hilfs- und Trägerstoffe In der Formulierung ausgewählt aus der Gruppe bestehend aus Phospholipiden, vorzugsweise Phosphoglyceride, Mizellstabllisatoren, vorzugsweise ausgewählt aus der Gruppe bestehend aus Carnitin oder/und einem physiologisch verträglichen Carnitin-Derivat oder/und Polyol(en), oder/und Radikalfängern, vorzugsweise ausgewählt aus der Gruppe bestehend aus Ascorbinsäure oder/und Alkall- oder/und Erdalkalisalze der Ascorbinsäure oder/und Ester einer oder mehrerer organischer Säuren mit Ascorbinsäure.

In einer weiteren bevorzugten Ausführungsform beträgt die Tagesdosis des Lektin's mindestens 2 - 100, vorzugsweise 10 - 50 mg.

Die Erfindung stellt somit eine Möglichkeit zur Verfügung Zellen, die für die Metastasierung verantwortlich oder daran beteiligt sind, für Abwehrzellen (Insbesondere natürliche Killer (NK)-Zellen) erkennbar zu machen. Weiterhin kann In einer bevorzugten Ausgestaltung der Erfindung das erforderliche Lektin in erhöhtem Masse an den betreffenden Ort Im Pateinten verfügbar gemacht werden.

In Laboruntersuchungen kann gezeigt werden, dass die zytotoxische Aktivität von NK-Zellen signifikant gesteigert wird durch z.B. Linsenlektin. Weitere Untersuchungen können demonstrieren, dass Karzinomzellen durch Inkubation mit z.B. Linsenlektin, das spezifisch an die Membrankohlenhydrate andockt, signifikant effektiver abgetötet werden können als native (unbehandelte) Kontroll-Karzlnomzellen. Die Erfinder verwenden somit vorteilhafter Weise und mit überraschendem Erfolg eine spezifische Anlagerung von großmolekularen Substraten (z. B. Linsenlektin) an Karzinomzellen zur Erkennung und verstärkten Abtötung dieser Zellen. Somit kann mittels der Erfindung das Immunsystem und Immunzellen von Krebspatienten mit einer erfindungsgemäßen Formulierung aktiviert werden und/oder es kann erreicht werden, dass der Körper effizienter gegen Metastase-bildende Zellen arbeiten kann.

2. Tertiäre Prophylaxe. Die Metastasierung von Karzinomen In Lunge und Leber ist meist Lebenszeit limitierend. Eine tertiäre Metastasen Prophylaxe ist einerseits möglich, wie unter primäre Prophylaxe oben beschrieben.
Ein zweiter erfolgversprechender Mechanismus Ansatz, den sich die Erfindung zu Nutze macht Ist wie folgt: Krebszellen präsentieren In Ihren Membranen spezifische Kohlenhydrate (z. B. N-Acetyl-Glucosamin/GlcNAc-Fucose/Fuc). Eine Metastasierung In Lunge und Leber erfolgt überwiegend über die spezifische Adhäsion von Karzinomzellen an Organzellen. Dabei adhäriert die Krebszelle über GlcNAc-Fuc Membrankohlenhydrate spezifisch an Organzellen, die GlcNAc-Fuc spezifische Lektine In ihrer Zellmembran aufweisen. Die Erfindung blockiert nun spezifisch die GlcNAc-Fuc Membrankohlenhydrate der Karzinomzellen mittels GlcNAc-Fuc spezifischem Linsenlektin. Damit kann der spezifische Erkennungsprozess verhindert werden, die Karzinomzellen können sich nicht an Organzellen anlagern und werden durch Abwehrsystem bzw. Scherkräfte während der Zirkulation Im Blut absterben.

Erfindungsgemäß kann die Krebstherapie optimiert werden und man kann somit von einer adjuvanten Metastasenprophylaxe mittels der Erfindung sprechen.

Die erfindungsgemäße pharmazeutische Formulierung umfasst alle für eine pharmazeutische Formulierung nötigen Hllfs- und Trägerstoffe, die dem Fachmann bekannt sind und somit hier nicht In Ihren Einzelheiten wiederholt werden zu brauchen.

Es kann jedwedes Lektin oder Lektingemisch verwendet werden und pharmazeutisch formuliert werden. Vorzugsweise umfasst die Formulierung ein oder mehrere Lektine.

Vorteilhafter Weise Ist das Lektin ein Erbsenlektin oder/und ein Linsenlektin.

Das Lektin kann In der erfindungsgemäßen Formulierung In jeder geeigneten Form vorliegen, die seine Wirkung nicht hemmt oder diese fördert. Vorteilhafter Welse liegt das oder die Lektine In mizellisierter Form vor.

Die Erfinder haben nunmehr überraschender Welse gefunden, dass Insbesondere Fuc-GlcNAc-spezifische Lektine, z. B. LCA/Linsenlektin und PSA/Erbsenlektin, besonders vorteilhaft zur Diagnostik und/oder Prophylaxe und/oder Behandlung von Krebs oder/und Verhinderung oder Verminderung der Metastasebildung bei Krebs oder/und zur Steigerung oder Induzierung der Immunabwehr oder/und zur Immunogenlsierung von Krebszellen verwendet werden können.

Die Erfinder konnten nun zeigen, dass mit der erfundungsgemäßen Formulierung viel versprechende innovative, lektinologische Prophylaxe- und Therapiekonzepte möglich gemacht werden können:
1. Durch Blockade von endständigem Fuc-GlcNAc auf Krebszellmembran Glykoproteinen durch spezifische Lektine (z. B. LCA oder/und PSA) kann die Anlagerung an andere Organzellen und somit die Metastasierung von Krebszellen gehemmt werden. Dies kann prophylaktisch/therapeutisch nutzbar gemacht werden, um nach Standardbehandlungen eine Metastasenprophylaxe durchzuführen.
2. Durch Anlagerung von Fuc-GlcNAc spezifischen Lektinen (z. B. LCA und/oder PSA) an Fuc-GlcNAc Rezeptoren auf Krebszellmembranen werden Krebszellen mit einem pflanzlichen Antigen besetzt und damit erkennbar und Immunologisch angreifbar gemacht. Insbesondere Krebszellen ohne eiweißhaltige Membranantigene können auf diese Weise dem Immunsystem (z. B. NK-Zellen, Lymphozyten, Makrophagen) effektiv präsentiert werden. Dies führt schließlich zum spezifischen Erkennen dieser Zellen und deren Abtötung.

Die Erfinder haben nunmehr durch experimentelle Laboruntersuchungen gezeigt, dass LCA-haltiger Linsenextrakt
1. die Krebszellen-abtötende Aktivität von NK-Zellen signifikant steigert;
2. den programmierten Krebszelltod (Apoptose) durch NK-Zellen signifikant steigert;
3. die Aktivität von Makrophagen zur Erkennung und Abtötung von Krebszellen (Phagozytose) signifikant steigert;
4. antioxidativ wirkt durch Reduktion des "oxidative burst".

Diese Datenlage zeigt den viel versprechenden und wirksamen prophylaktischen Wert der spezifischen Anwendung von pflanzlichen Lektinen, vorzugsweise LCA und/oder PSA, nach der Erfindung.

Die Erfindung macht sich weiterhin den Umstand zu Nutze, dass sich Reifegrad, Wachstumsverhalten und Aufbau der Zellmembranen von Krebszellen meist stark von Normalzellen unterscheiden. Insbesondere die genetisch festgelegten Bestandteile der Zellmembranen (z. B. Proteine, Kohlenhydratketten) unterscheiden sich oft gravierend. Während Normalzellen durch ihre Membranproteine und - Kohlenhydrate ihre spezifische Antigenität entfalten, sind diese Moleküle In Krebszellen stark verändert oder fehlen. Fehlende Erkennbarkelt von Krebszellmembranantigenen (entweder durch deren Masklerung mit körpereigenen Eiweißen oder durch deren Fehlen) führt zwangsläufig zur Unwirksamkeit spezifischer Immunonkologischer Theraplekonzepte. Die Erfinder haben nunmehr eine Form der innovativen Immunonkologie entwickelt und Moleküle bereitgestellt, die sich als Antigene an die veränderten Zellmembranen anlagern und diese für das Immunsystem erkennbar machen. Dadurch können vorteilhafte Ergebnisse In Behandlung und Prophylaxe sowie Metastaseverhinderung oder -verminderung oder -verzögerung erreicht werden.

Die therapeutischen Effekte der erfindungsgemäßen Formulierung können weiter signifikant verstärkt werden, z. B durch deren Aufbereitung mittels liposomaler Verkapselung oder Mizellisierung. Insbesondere die Mizellisierung, d. h. die Umhüllung von Lektinen mit Kohlenhydrat-Eiweiß- oder Kohlenhydrat-Fett-Gemischen (Glykoproteinen oder Glykolipiden), verbessert deren Resorption und deren prophylaktische und therapeutische Wirksamkeit.

Die Erfinder haben somit eine therapeutische Möglichkeit bereitgestellt, die hochspezifisch und wirksam ist, um Metastasen zu vermeiden oder deren Bildung zumindest zu verzögern bzw. die zur Metastaseprophylaxe (Primär- bis Tertiärprophylaxe) geeignet Ist.

### Beispiele

### 1. NK-Zellfunktionstest

Mit dieser Immunologischen Standardmethode wird die NK (Natürliche Killer) Zell Zytotoxizität unter Einfluss von erfindungsgemäßen Formulierungen (umfassend z. B. *Lens culinarius* Agglutinin, LCA/Linsenlektin, *Pisum sativum* Agglutinin, PSA/Erbsenlektin sowie lektinhaltiger Linsen- oder Erbsenextrakte) untersucht.

### Testprinzip

Inkubation von Patientenzellen (oder Tumorzellen) mit einer erfindungsgemäßen Formulierung und deren Verwendung umfassend LCA, PSA oder lektinhaltigen Linsen- oder Erbsenextrakten (Immunmodulatoren), anschließend Messung der zytotoxischen Aktivität gegenüber K 562 Tumorzellen.

### Probenmaterial

Heparin-Blut (ca. 20 ml, maximal 24 h haltbar) bel Raumtemperatur (RT).

### Probenvorbereitung

Material: möglichst 2 heparinbeschichtete Monovetten.
- Flcolltrennung (in Anlehnung an Formblätter SO/Standard Operating Procedure; NK-Zellplatz)
- Patientenzellen auszählen (siehe SOP-MU-IMM.M.0009.xx) und auf 1 x 10⁶/ml einstellen:
- Verdünnungsreihe der benötigten Testsubstanzen (LCA, PSA, lektinhaltige Extrakte) herstellen:
- Stimulationsansatz In FACS (Fluorescence-Actlvated Cell Sorter)-Rörchen
   Basal: 900 µl Medium + 100 µl Patientenzellsuspension
   IL2 positive Kontrolle: 900 µl Medium + 100 µl Patientenzellsuspension + 10µl IL2
   Testsubstanzen (mit Konzentrationskinetik/Verdünnungen):
      800 µl Medium + 100 µl Patientenzellsuspension (+ 100 µl Tumorzellsuspension) + 100 µl Testsubstanz
- Röhrchen über Nacht bel 37 Grad + 5 % CO2 Im Brutschrank (IMMUTOX) inkubleren
- Ansatz für (%) NK-Zellantellbestimmung:
   - 10 µl Antikörper (CD3/ CD16+CD56) In FACS-Röhrchen vorlegen
   - 100 µl Patientenzellsuspension (Tumorzellsuspension) zugeben
   - 15 Min. bei RT Inkubieren
   - 100 µl Flow Count Beads (gevortext/aufgeschwemmt; vor Gebrauch nochmals gemischt) + ca. 1 ml PBS (Phosphate Buffered Sallne/Phosphatpufferlösung) zugeben
   - am FACS Scan (FS) 500, unter Protokoll (Resource Explorer) NK-Zellen Flow Count messen.
   - % Angabe der NK-Zellen ermittelt und ausgedruckt.
- Über Nacht kultivierte Zellsuspensionen (1 ml) 10 Min. bei 800 UpM zentrifugieren und dekantieren.
- Gefärbte K 562 Tumorzellen fugen (10 min., 500UpM), dekantieren und mit 1 ml K562-Medium auffüllen und auf 1 x 10^5 einstellen (In Anlehnung an entsprechende SOP)
- Berechnung benötigter K562-Zellmenge (Im Verhältnis 1:1 mit den absoluten NK-Zellen des Probanden). Absolut-Wert wird der Spalte Count beim % NK-Zellantell entnommen z.B. C1= 5% und Count= 115
- Zum Probanden-Zellpellet K 562 Tumorzellen hinzu pipettieren (siehe Berechnung benötigter K562-Zellmenge) z. B. 115µl (aber mindestens 100µl bis max. 300µl)
- Ein Röhrchen mit mind, 200µl K562 Tumorzellen und mind, 200µl Medium mitführen (K 562 Kontrolle)
- Alle Röhrchen 3 - 4 Min. bel ca. 500 UpM zentrifugieren (ohne Bremse)
- Röhrchen mit Aluminiumfolle abdecken und 2 Stunden bel 37°C im Brutschrank Inkubieren danach auf Eis stellen.
- 100 µl DNA - Färbelösung (Propidiumjodidlösung) zum Anfärben der DNA In jedes Röhrchen pipettieren und vortexen, 10 Min. im Dunkeln auf Eis stehen lassen und Innerhalb von 30 Min. mittels FS 500 messen

### 2. Messung am Durchflußzytometer

Referenzbereich (gemessen als % markierter bzw. aktivierter) Zellen Basal ist die Ausgangsaktivität von NK Zellen ohne Stimulation IL2 (Interleukin-2) Ist einer der stärksten Immunologischen Aktivatoren von NK Zellen und wird als Positivkontrolle betrachtet. Diese Werte deuten auf die optimale Aktivlerungskapazität von NK Zellen hin.

Einer der genannten Testansätze wurde bereits durchgeführt und belegt eine signifikant gesteigerte NK Zell Aktivität durch Tumorzellen, wenn die NK Zellen mit Il-2 bzw. diversen erfindungsgemäßen Formullerungen (L = Linsenlektin) geprimt waren. Im Test werden NK-Zellen mit Testsubstanzen Inkubiert und anschileßend mit Tumorzellen aktiviert. Die NK Zell Aktivierung ist vergleichbar mit der Aktivierung durch IL-2, was auf eine starke Aktivierungskapazität hinweist.

| **% markierter bzw. aktivierter NK Zellen** | | | | | | |
|---|---|---|---|---|---|---|
| **Probenden** | **Basal** | **Il-2** | **L1** | **L2** | **L3** | **L4** |
| **1** | **4.4** | **25.1** | **25.5** | **28.8** | **28.7** | **28.7** |
| **2** | **4.8** | **11.1** | **12.4** | **L2.4** | **18.8** | **13.8** |
| **3** | **4.6** | **27.2** | **23.8** | **22.3** | **25.5** | **28.2** |
| **4** | **5.5** | **10.5** | **10.5** | **12.0** | **10.4** | **13.2** |

3. In einem Testversuch (Aufbau wie oben beschrieben) werden die Tumorzellen mit den Testsubstanzen (erfindungsgemäßen Formulierungen) Inkubiert, um deren Antigenität zu steigern.

In diesem labortechnischen Aufbau kann die erfindungsgemäße Formulierung bzw. deren Verwendung eingesetzt werden und gezeigt werden, dass durch eine Markierung der Tumorzellen mit LCA, PSA oder lektinhaltiger Linsen- oder Erbsenextrakte die NK Zell Zytotoxizität gegen die antigenmarkierten Tumorzellen signifikant erhöht werden kann.

### 3. Organzellen und Tumorzellen Im Rosetten Test

### Kontrollansatz

Menschliche vitale, adhärente Tumorzellen (z. B. Mammakarzinom MDA-231; Fibrosarkorn HT-1080; Zervixkarzinom HeLa; je 1 x 10⁷ pro ml MEM (Minimal Essential Medium), dem 10% Ultraser G und 2mM Glutamin beigefügt werden) werden für 12 Stunden bei 37 Grad Celsius in 5% CO₂-haltiger Atmosphäre In Mikrotlterplatten Inkubiert und Imponleren als nicht konflulerende Monolayer. Die adhärenten Tumorzellen werden mit einer Organzellsuspension (z. B. vitale Lungenzellen, Leberzellen; 1 x 10⁷ Zellen pro ml Medium (MEM) überschichtet und eine Stunde bei 37 Grad in 5% CO₂-haltiger Atmosphäre Inkubiert. Der Überstand wird dekantiert, die Mlkrotiterplatten werden dreimal mit Phosphatpuffer (PBS) gewaschen und die Anzahl der Tumor-/Organzellrosetten mikroskopisch ausgezählt (Kontrollansatz).

### Testansatz

Rosetten Inhibitions Tests erfolgen In Anwesenheit von *Lens culinaris* Agglutinin (LCA, 1 mg; 0.5 mg; 0.05 mg pro ml Medium). Die inhibitorische Aktivität von LCA wird In zwei Ansätzen getestet durch Zugabe 1, in das Zellkulturmedium der adhärierenden Tumorzellen; 2. In das Zellkulturmedium der Organzellen. Die adhärierenden Tumorzellen und die Organzellen werden für eine Stunde In LCAhaltlgem Medium Inkubiert und anschließend für eine weitere Stunde wie folgt überschichtet: LCA-Inkubierte Tumorzellen + Organzellen (Testansatz 1); Tumorzellen + LCA-Inkubierte Organzellen Testansatz 2). Anschließend wird der Überstand dekantiert, die Mikrotiterplatten mit Phosphatpuffer (PBS) gewaschen (dreimal) und die Anzahl der Tumor-/Organzellrosetten mikroskopisch ausgezählt (Netland PA, Zettier BR, Organ-specific adhesion of metastatic tumor cells In vitro, Science 224,1113-1115,1984; Schirrmacher V et al., Hepatocytetumor cell interaction In vitro, Conditions for rosette formation and Inhibition by anti-H-2-antibody, J Exp Med 151,984-989, 1980).

### Ergebnis

Nach Auswertung der Versuchsansätze mit Präinkubation mit Planzenlektin (z.B. Lens culinaris) und der Kontrollansätze zeigt sich eine verminderte Rosettenbildung in den mit Pflanzenlektin präinkubierten Ansätzen.

Die verminderte Rosettenbildung kann so erklärt werden, dass das Planzenlektin spezifisch an die Tumorzellen andockt und somit ein Interaktion der Tumorzellen mit Organzellen verhindert wird. Die gesunden Organzellen werden quasi geschützt und somit die Matastasenbildung entweder ganz verhindert oder zumindest erheblich vermindert oder jedenfalls wird eine Metastasenbildung verzögert.

Technisch gesehen wird es mit der Erfindung möglich nun spezifisch die Adhäsionsmoleküle der Karzinomzellen mittels GlcNAc-Fuc spezifischem Linsenlektin zu blockieren.

Damit kann die Anlagerung der Krebszelle an die Organzelle vermindert oder ganz verhindert werden, was zu weniger Rosettenformation führt und dadurch nachweisbar ist.

Die Auszählung kann mittels mikroskopischer Auszählung oder auch mittels Isotopenmarkierung erfolgen. Die entsprechenden Verfahren sind dem Fachmann bekannt und müssen deshalb hier nicht In allen Details wiederholt werden.

## Patentansprüche

1. Pharmazeutische Formulierung umfassend ein oder mehrere Lektine zur Verwendung bel der Prophylaxe oder/und der Verhinderung oder Verminderung der Metastasenbildung bel Krebspatienten oder/und zur Steigerung oder Induzierung der Immunabwehr oder/und zur immunogenislerung von Krebszellen, wobei der Krebs ein Karzinom Ist, wobei das Lektin ein Erbsenlektin oder/und ein Linsenlektin Ist, wobei das oder die Lektine in mizellisierter Form vorliegen.

2. Pharmazeutische Formulierung zur Verwendung nach Anspruch 1, wobei Karzinome Brust-, Darm-, Prostata- oder Lungenkarzinome sind.

3. Pharmazeutische Formulierung zur Verwendung nach Anspruch 1 oder 2 umfassend gegebenenfalls weitere Hilfs- und Trägerstoffe.

4. Pharmazeutische Formulierung zur Verwendung nach Anspruch 1, 2 oder 3, wobei das Lektin ein oder mehrere Lektine umfasst.

## Claims

1. A pharmaceutical formulation comprising one or more lectins for use in the prophylaxis or/and the prevention or reduction of metastasis in cancer patients or/and for increasing or inducing immune defence or/and for immunogenising cancer cells, wherein the cancer is a carcinoma, wherein the lectin is a pea lectin or/and a lens lectin, wherein the lectin or lectins are present in micellised form.

2. A pharmaceutical formulation for use according to claim 1, wherein carcinomas are breast, colon, prostate or lung carcinomas.

3. A pharmaceutical formulation for use according to claim 1 or 2, optionally comprising further excipients and carriers.

4. A pharmaceutical formulation for use according to claim 1, 2 or 3, wherein the lectin comprises one or more lectins.

## Revendications

1. Formulation pharmaceutique comprenant une ou plusieurs lectines et destinée à être utilisée dans la prophylaxie et/ou la prévention ou la réduction des métastases chez les patients cancéreux et/ou pour augmenter ou induire la défense immunitaire et/ou pour immunogénéiser les cellules cancéreuses, le cancer étant un carcinome, la lectine étant une lectine de pois et/ou une lectine de lentille, et la ou les lectines étant sous forme micellaire.

2. Formulation pharmaceutique pour une utilisation selon la revendication 1, les carcinomes étant des carcinomes du sein, du colon, de la prostate ou du poumon.

3. Formulation pharmaceutique pour une utilisation selon la revendication 1 ou 2, comprenant éventuellement d'autres excipients et supports.

4. Formulation pharmaceutique pour une utilisation selon la revendication 1, 2 ou 3, dans laquelle la lectine comprend une ou plusieurs lectines.
